(19) Europäisches Patentamt
European Patent Office
Office européen des brevets

(11) Veröffentlichungsnummer: **0 085 893**
**B1**

(12) # EUROPÄISCHE PATENTSCHRIFT

(45) Veröffentlichungstag der Patentschrift: **14.05.86**

(21) Anmeldenummer: **83100679.6**

(22) Anmeldetag: **26.01.83**

(51) Int. Cl.⁴: **C 07 D 471/04,** C 07 D 519/00, A 61 K 31/55 // (C07D471/04, 243:00, 221:00)

(54) **Pyridobenzodiazepinone, Verfahren zu ihrer Herstellung und diese Verbindungen enthaltende Arzneimittel.**

(30) Priorität: **09.02.82 DE 3204401**

(43) Veröffentlichungstag der Anmeldung: **17.08.83 Patentblatt 83/33**

(45) Bekanntmachung des Hinweises auf die Patenterteilung: **14.05.86 Patentblatt 86/20**

(84) Benannte Vertragsstaaten: **AT BE CH DE FR GB IT LI LU NL SE**

(56) Entgegenhaltungen:
**DE - A - 2 644 121**
**DE - A - 2 724 434**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Engel, Wolfhard, Dr., Dipl.-Chem., Mozartstrasse 13, D-7950 Biberach 1 (DE)**
Erfinder: **Schmidt, Günther, Dr., Dipl.-Chem., Johann-Seb.-Bach-Strasse 27, D-7950 Biberach 1 (DE)**
Erfinder: **Trummlitz, Günter, Dr., Dipl.-Chem., Buchenweg 27, D-7951 Warthausen (DE)**
Erfinder: **Eberlein, Wolfgang, Dr., Dipl.-Chem., Obere Au 6, D-7950 Biberach 1 (DE)**
Erfinder: **Hammer, Rudolf, Dr., Via Fabio Filzi 33, Milano (IT)**
Erfinder: **Del Soldato, Piero, Dr., Via E. Toti 22, Monza (IT)**

**EP 0 085 893 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In den US-Patentschriften 3 660 380, 3 691 159, 4 213 984, 4 213 985 und 4 210 648 werden bereits Pyridobenzodiazepinone mit ulcus-hemmenden und sekretionshemmenden Eigenschaften beschrieben.

Es wurde nun gefunden, dass die neuen Pyridobenzodiazepinone mit neuartigen Aminoacylresten gegenüber den oben erwähnten Verbindungen interessante und überlegene pharmakologische Eigenschaften aufweisen.

Gegenstand der Erfindung sind somit die neuen Pyridobenzodiazepinone der allgemeinen Formel I

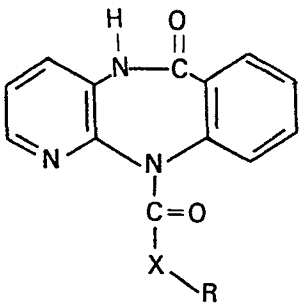

(I)

in der

X ein Sauerstoffatom, eine –NH– oder $-NCH_3$-Gruppe und

R einen gegebenenfalls durch eine Methylgruppe substituierten 1-Methyl -4- piperidinyl-, 4-Methyl -1- piperazinyl-, 3α- oder 3β-Tropanyl-Rest bedeuten, sowie ihre Säureadditionssalze.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Citronensäure, Maleinsäure, Bernsteinsäure, Gluconsäure, Apfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

5,11-Dihydro-11-[[(1-methyl -4- piperidinyl)-oxy]carbonyl]- 6H-pyrido[2,3-b] [1,4]benzodiazepin -6- on,

cis-5,11-Dihydro-11-[[(1,2-dimethyl -4- piperidinyl)oxy]carbonyl]-6H-pyrido[2,3-b][1,4]benzodiazepin -6- on,

trans-5,11-Dihydro-11-[[(1,2-dimethyl -4-piperidinyl)oxy]carbonyl]- 6H-pyrido[2,3-b][1,4] benzodiazepin -6- on,

cis-5,11-Dihydro-11-[[(1,3-dimethyl -4- piperidinyl)oxy]carbonyl]-6H-pyrido[2,3-b][1,4]benzodiazepin -6- on,

trans-5,11-Dihydro-11-[[(1,3-dimethyl-4-piperidinyl)oxy]carbonyl]- 6H-pyrido[2,3-b][1,4] benzodiazepin -6- on,

5,11-Dihydro-5-[[(1-methyl -4- piperidinyl)-amino]carbonyl]- 6H-pyrido[2,3-b][1,4]benzodiazepin -6- on,

cis-5,11-Dihydro-11-[[(1,2-dimethyl -4- piperidinyl)amino]carbonyl]- 6H-pyrido[2,3-b][1,4] benzodiazepin -6- on,

trans-5,11-Dihydro- 11-[[(1,2-dimethyl-4-piperidinyl)amino]carbonyl]- 6H-pyrido[2,3-b][1,4] benzodiazepin -6- on,

cis-5,11-Dihydro-11-[[(1,3-dimethyl -4- piperidinyl)amino]carbonyl]- 6H-pyrido[2,3-b][1,4] benzodiazepin -6- on,

trans-5,11-Dihydro- 11-[[(1,3-dimethyl-4-piperidinyl)amino]carbonyl]- 6H-pyrido[2,3-b][1,4] benzodiazepin -6- on,

5,11-Dihydro-11-[[(4-methyl -1- piperazinyl)-amino]carbonyl] -6H-pyrido[2,3-b][1,4]benzodiazepin -6- on,

5,11-Dihydro-11- [[(3,4-dimethyl -1- piperazinyl)amino]carbonyl]-6H-pyrido[2,3-b][1,4]-benzodiazepin -6- on,

5,11-Dihydro-11- [[(2,4-dimethyl -1- piperazinyl)amino]carbonyl]-6H-pyrido[2,3-b][1,4]-benzodiazepin -6- on,

5,11-Dihydro-11- [[(4-methyl -1- piperazinyl)-oxy]carbonyl]- 6H-pyrido[2,3-b] [1,4]benzodiazepin -6- on,

5,11-Dihydro-11- [[(3,4-dimethyl -1- piperazinyl)oxy]carbonyl]- 6H-pyrido [2,3-b] [1,4]-benzodiazepin -6- on,

5,11-Dihydro-11- [[(2,4-dimethyl -1- piperazinyl)oxy]carbonyl]- 6H-pyrido [2,3-b] [1,4]-benzodiazepin -6- on,

endo-5,11-Dihydro-11-[[(8-methyl -8- azabicyclo[3,2,1]oct -3- yl)-oxy]-carbonyl]-6H-pyrido[2,3-b][1,4]benzodiazepin-6-on,

exo 5,11-Dihydro-11-[[(8-methyl -8- azabicyclo[3,2,1]oct -3- yl)-oxy]-carbonyl]-6H-pyrido[2,3-b][1,4]benzodiazepin -6- on,

endo-5,11-Dihydro-11-[[(8-methyl -8- azabicyclo[3,2,1]oct -3- yl)-oxy]carbonyl]-6H-pyrido[2,3-b][1,4]benzodiazepin -6- on,

exo-5,11-Dihydro-11-[[(8-methyl -8- azabicyclo[3,2,1]oct -3- yl)-amino]carbonyl]-6H-pyrido[2,3-b][1,4]benzodiazepin -6- on,

5,11-Dihydro-11-[[N-methyl-N-(1-methyl -4-piperidinyl)amino]carbonyl]- 6H-pyrido[2,3-b]-[1,4]benzodiazepin -6- on,

cis-5,11- Dihydro-11- [[N-methyl-N- (1,2-dimethyl -4- piperidinyl)-amino]carbonyl]-6H-pyrido[2,3-b][1,4]benzodiazepin -6- on,

trans-5,11-Dihydro-11- [[N-methyl-N-(1,2-dimethyl -4- piperidinyl)-amino]carbonyl]-6H-pyrido[2,3-b][1,4]benzodiazepin -6- on,

cis-5,11- Dihydro-11- [[N-methyl-N- (1,3-dimethyl -4- piperidinyl)-amino]carbonyl]-6H-pyrido[2,3-b][1,4]benzodiazepin -6- on,

trans-5,11-Dihydro-11- [[N-methyl-N-(1,3-dimethyl -4- piperidinyl)-amino]carbonyl]-6H-pyrido[2,3-b][1,4]benzodiazepin -6- on,

5,11-Dihydro-11- [[N-methyl-(4-methyl -1- piperazinyl)amino]carbonyl]- 6H- pyrido [2,3-b]-[1,4]benzodiazepin -6- on,

5,11-Dihydro-11-[[N-methyl-(3,4-dimethyl -1-piperazinyl)amino]carbonyl]- 6H-pyrido[2,3-b]-[1,4]benzodiazepin -6- on,

5,11-Dihydro-11-[[N-methyl-(2,4-dimethyl -1-

piperazinyl)amino]carbonyl]-6H-pyrido[2,3-b]-[1,4]benzodiazepin-6-on.

Gegenstand der Erfindung sind darüber hinaus die als Zwischenprodukte verwendeten Pyridobenzodiazepinone der allgemeinen Formel Ia

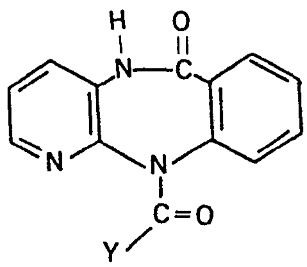

(Ia)

in der

Y ein Halogenatom, bevorzugt ein Brom- oder Chloratom, oder den Rest $OR_1$ bedeutet, wobei $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt.

$R_1$ besitzt somit beispielsweise die Bedeutung der Methyl-, Ethyl-, n-Butyl-, Isobutyl-, Benzyl-, 9-Fluorenylmethyl-, Phenyl-, 4-Nitrophenyl-, 2,2,2-Trichlorethyl-, 2,4,5-Trichlorphenyl- oder 2,2,2-Trichlor-tert.-butyl-Gruppe.

Ein weiterer Gegenstand der Erfindung sind Arzneimittel, die ein oder mehrere Pyridobenzodiazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragees, Kapseln oder Teezubereitungen einarbeiten. Die Tagesdosis liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben zur Erzielung der gewünschten Ergebnisse verabreicht wird.

Die substituierten Pyridobenzodiazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet; sie hemmen z.B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der substituierten Pyridobenzodiazepinone der allgemeinen Formel I und ihrer pharmakologisch, d.h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise können mit ihnen akute und chronische Ulcera ventriculi und duodeni, Gastritis und hyperacider Reizmagen bei Mensch und Tier behandelt werden.

Sollen die erfindungsgemässen substituierten Pyridobenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Sekretionshemmer, wie $H_2$-Blocker, z.B. Cimetidin, Ranitidin; Magen- und Darmtherapeutika, z.B. Metoclopramid, Bromoprid, Tiaprid; Tranquilizer, wie Benzodiazepine, beispielsweise Diazepam, Oxazepam; Spasmolytika, z.B. Bietamiverin, Camylofin; Anticholinergica, z.B. Oxyphencyclimin, Phencarbamid; Glucocorticoide, wie Prednisolon, Fluocortolon, Betamethason; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z.B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon; Lokalanästhetika, beispielsweise Tetracain, Procain; gegebenenfalls auch Fermente, Vitamine, Aminosäuren etc. enthalten.

Erfindungsgemäss erhält man die neuen Pyridobenzodiazepinone der allgemeinen Formel I nach folgenden Verfahren:

a) Umsetzung eines Pyridobenzodiazepinons der allgemeinen Formel Ia

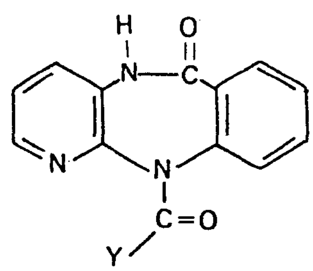

(Ia)

in der

Y ein Halogenatom, bevorzugt ein Brom- oder Chloratom, oder den Rest $OR_1$ bedeutet, wobei $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, werden mit Verbindungen der allgemeinen Formel II

H–X–R (II)

in der

X und R die eingangs angegebenen Bedeutungen haben, umgesetzt.

Die Umsetzung wird ohne oder bevorzugt in Gegenwart inerter Lösungsmittel, z.B. von Wasser, Toluol oder von Alkoholen, wie z.B. Methanol, Ethanol oder Isopropanol, ganz bevorzugt jedoch in Gegenwart aprotischer polarer Lösungsmittel, z.B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen 0 °C und dem Siedepunkt des Reaktionsgemisches, bevorzugt zwischen 40 und 100 °C durchgeführt. Bewährt hat sich die Verwendung zusätzlicher anorganischer oder organischer Basen, z.B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat; von tertiären Aminen, z.B. von Triethylamin, Ethyldiisopropylamin, N,N-Dimethylanilin oder von Pyridin; sowie die Umsetzung in Gegenwart eines Überschusses einer Verbindung der allgemeinen Formel II.

Die Umsetzung kann jedoch auch mit einer Metallverbindung der allgemeinen Formel IIa

M–X–R (IIa)

in der

M ein Alkalimetallatom oder 1 Äquivalent eines Erdalkalimetallatoms bedeutet, durchgeführt werden. Dabei lassen sich Metallverbindungen der allgemeinen Formel IIa aus II durch Umsetzung mit Alkali- oder Erdalkalimetallen, etwa mit Natrium, Kalium oder Barium, oder mit Alkali- oder Erdalkalihydriden, etwa mit Natrium-, Kalium- oder Calciumhydrid, oder durch Reaktion mit Alkali- oder Erdalkaliorganylen, z.B. mit n-Butyl-lithium oder Phenyllithium, in situ leicht herstellen.

b) Umsetzung von 5,11-Dihydro-6H-pyrido[2,3-b][1,4]benzodiazepin -6- on mit einem Chlorkohlensäurederivat der allgemeinen Formel IV

```
Cl–C–X–R          (IV)
   ||
   O
```

oder mit einem Isocyanat der allgemeinen Formel IVa

O =C =N–R (IVa)

in der

R und X wie oben definiert sind.

Die Umsetzung wird vorzugsweise in inerten organischen Lösungsmitteln, beispielsweise in aromatischen Kohlenwasserstoffen wie Toluol, Xylol, in Äthern wie Diisopropyläther, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen +30 und +100 °C, durchgeführt.

So erhaltene Basen der allgemeinen Formel I können anschliessend in ihre Säureadditionssalze oder erhaltene Säureadditionssalze in die freien Basen oder andere pharmakologisch verträgliche Säureadditionssalze übergeführt werden.

Ein Teil der erfindungsgemässen Pyridobenzodiazepinone der allgemeinen Formel I enthält ein oder zwei asymmetrische Kohlenstoffatome in der Seitenkette –CO–X–R. Diese Verbindungen können deshalb in zwei diastereomeren cis- und trans-Formen oder jeweils als enantiomere (+)- und (–)-Formen auftreten. Die Erfindung umfasst die einzelnen Isomeren ebenso wie ihre Gemische.

Die Trennung der jeweiligen Diastereomeren gelingt auf Grund der unterschiedlichen physikochemischen Eigenschaften, z.B. durch fraktioniertes Umkristallisieren aus geeigneten Lösungsmitteln, durch Hochdruckflüssigkeitschromatographie oder gaschromatographische Verfahren. Jeweils nur ein Diastereomeres wird erhalten, wenn man die oben beschriebenen Synthesen mit nur einem Diastereomeren der allgemeinen Formel II bzw. IIa durchführt.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (–)-Weinsäure, oder eines Derivats davon, wie (+)- oder (–)-Diacetylweinsäure, (+)- oder (–)-Monomethyltartrat oder (+)-Camphersulfonsäure.

Nach einem üblichen Verfahren zur Isomerentrennung wird das Razemat einer Verbindung der allgemeinen Formel I mit einer der vorstehend angegebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (–)-Form erhalten.

Jeweils nur ein Enantiomeres wird auch dadurch erhalten, dass man die oben beschriebenen Synthesen mit nur einem Enantiomeren der allgemeinen Formel II bzw. IIa durchführt.

Die als Zwischenprodukte erforderlichen neuen Verbindungen der allgemeinen Formel Ia erhält man erfindungsgemäss durch Umsetzung des literaturbekannten 5,11-Dihydro-6H-pyrido[2,3-b][1,4]-benzodiazepin -6- on (siehe US-Patent 3 406 168) mit einem Halogenkohlensäurederivat der allgemeinen Formel III

```
Hal–C–Y          (III)
    ||
    O
```

in der

Hal ein Brom- oder Chloratom, bevorzugt ein Chloratom, bedeutet und Y die oben angegebenen Bedeutungen hat.

Die Reaktion wird in inerten organischen Lösungsmitteln, beispielsweise aromatischen Kohlenwasserstoffen, wie Toluol, Chlorbenzol oder Xylol; offenkettigen oder cyclischen Ethern, wie Diisopropylether, Tetrahydrofuran oder Dioxan; offenkettigen oder cyclischen aliphatischen Ketonen, beispielsweise 3-Pentanon; chlorierten aliphatischen Kohlenwasserstoffen, wie 1,2-Dichlorethan oder anderen Lösungsmitteln, wie Acetonitril oder Dimethylformamid oder in Gemischen davon und bevorzugt in Gegenwart tertiärer organischer Basen, bevorzugt von Pyridin, und bei Temperaturen bis höchstens zum Siedepunkt des verwendeten Lösungsmittels oder Lösungsmittelgemisches, bevorzugt zwischen +30 und +80 °C, durchgeführt.

Die Ausgangsverbindungen der allgemeinen Formel II sind bekannt oder können in Analogie zu literaturbekannten Verfahren bereitet werden. Beispielsweise erhält man 1-Hydroxy -4- methylpiperazin nach S.M. Riba, A.S. Issa und Y.A. Beltagy, Pharmazie 33, 711 (1978) durch Umsetzung von Bis[N-(2-Chlor-ethyl)]methylamin mit Hydroxylaminhydrochlorid in wässrig-ethanolischer Lösung und in Gegenwart von Kaliumcarbonat, 3α-Aminotropan und 3α-Methylaminotropan nach S. Archer u.a., J. Amer. Chem. Soc. 79, 4194–4198 (1957); 3β-Aminotropan nach R. Willstätter u.a., Ber. dtsch. chem. Ges. 31, 1202 (1898); Pseudotropin nach J.J. Tufariello u.a., J. Amer. Chem. Soc. 101, 2435–2442 (1979).

Halogenkohlensäurederivate der allgemeinen Formel III sind bekannt.

Chlorkohlensäurederivate der allgemeinen Formel IV und Isocyanate der allgemeinen Formel IVa sind bekannt oder können in Analogie zu literaturbekannten Verfahren erhalten werden (siehe beispielsweise I.W. Mathison u.a., J. Pharm. Sci. 62, 158 [1963]; H. Hopff und H. Ohlinger, Angew. Chem. 61, 183 [1949]; W. Siefken, Liebigs Ann. Chem. 562, 75 [1949]; Houben-Weyl VIII, 117; Ullmann V, 72; L.C. Raiford und K. Alexander, J. Org. Chem. 5, 306 [1940]; H.H. Saunders und R.J. Slocombe, Chem. Rev. 43, 203 [1948]; R.J. Slocombe, E.E. Hardy, J.H. Saunders und R.L. Jenkins, J. Amer. chem. Soc. 72, 1888 [1950]; H. Habad und A.G. Zeiler, Chem. Rev. 73, 75 [1973].

Wie bereits oben erwähnt, weisen die neuen Verbindungen der allgemeinen Formel I wertvolle pharmakologische Eigenschaften auf; insbesondere besitzen sie antiulcerogene und magensäuresekretionshemmende Wirkungen, sowie günstige Effekte auf verschiedene andere Erkrankungen des Magen-Darm-Traktes, unter denen das Colon irritabile besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen,dass die erfindungsgemässen Verbindungen diesbezüglich überraschend günstige Relationen aufweisen.

Untersuchung auf Selektivität der antimuscarinischen Wirkung

Ziel:

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik:

Verwendet wurden 10 weibliche Albino-Ratten (Stamm Crl:COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100% der Tiere ausgelöst hatte.

Magenschleimhaut-Läsionen: 0,62 mg/kg i.v.
Speichelsekretion: 0,083 mg/kg i.v.

Jede antimuscarinische Substanz wurde in gleichmässig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d.h. der Untersucher wusste nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. 96, 99, 1949) ermittelt.

Die Effekte auf Schleimhaut-Läsionen des Magens wurden folgendermassen bewertet:

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung

der Prüfsubstanzen wurde als prozentuale Hemmung (Prozentsatz der Tiere ohne Läsionen) ausgedrückt. $ED_{50}$- bzw. $ED_{70}$-Werte wurden nach der Methode von LITCHFIELD und WILCOXON (s.o.) ermittelt.

Mydriasis

Der Effekt von Testsubstanzen auf die Pupillenweite von Ratten wurde folgendermassen untersucht:

Die Substanzen wurden an Gruppen von jeweils 10 Tieren in wenigstens 3 gleichmässig abgestuften Dosierungen intravenös appliziert. Die Pupillenweite wurde anschliessend 10 Minuten lang auf evtl. Veränderungen (auftreten von Mydriasis oder Miosis) beobachtet, und zwar wiederum blind, d.h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s.o.) bestimmt.

2. Bindungsstudien an muscarinischen Rezeptoren:

Bestimmung des $IC_{50}$-Wertes

Als Organspender dienten männliche Sprague-Dawley-Ratten mit 180–220 g Körpergewicht. Nach Entnahme von Herz, Magen und Grosshirnrinde wurden alle weiteren Schritte im eiskalten Hepes-HCl-Puffer (pH 7,4; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschliessend in einem Potter homogenisiert.

Für den Bindungstest wurden die Organhomogenate in folgender Weise verdünnt:

| | |
|---|---|
| Glatter Muskel Magenfundus | 1:100 |
| Gesamtherz | 1:250 |
| Grosshirnrinde | 1:3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3n molar $^3H$–N-Methylscopolamin ($^3H$–NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3H$–NMS. Der Anteil der unspezifischen Bindung wurde definiert als jene Radioaktivität, die in Anwesenheit von 1 μ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3H$–NMS an die muscarinischen Rezeptoren in den verschiedenen Organen um 50% gehemmt wurde.

Nach den vorstehenden Angaben wurden beispielsweise die folgenden Verbindungen untersucht:

A = 5,11-Dihydro-11-[[(1-methyl -4- piperidinyl)oxy]carbonyl]-6H-pyrido[2,3-b][1,4]benzodiazepin -6- on,

B = 5,11-Dihydro-11-[[(1-methyl -4- piperidinyl)amino]carbonyl]- 6H-pyrido[2,3-b][1,4] benzodiazepin -6- on und

C = 5,11-Dihydro-11-[[(4-methyl -1- piperazinyl)amino]carbonyl]-6H-pyrido[2,3-b][1,4]-benzodiazepin -6- on.

Ergebnisse

| Substanz | Rezeptor-Bindungs-Tests $IC_{50}$ [n Mol $1^{-1}$] Cortex | Glatter Muskel-Magenfundus | Herz | Oxotremorin-Tests [ug/kg] i.v. Antiulcerogene Wirkung $ED_{50}$ | $ED_{70}$ | Salivationshemmung $ED_{50}$ | Mydriasis $ED_{50}$ [μg/kg] i.v. |
|---|---|---|---|---|---|---|---|
| A | 100 | ca. 900 | 500 | 10 | 30 | 850 | 130 |
| B | 200 | 1 200 | | 6,8 | 19 | 120 | 139 |
| C | 60 | 900 | | 3,3 | 5,2 | 56 | 70 |

Die Angaben in der vorstehenden Tabelle beweisen, dass die genannten Verbindungen generell eine hohe Affinität zu muscarinischen Rezeptoren besitzen. Darüber hinaus kann man den Daten entnehmen, dass die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Grosshirnrinde gegenüber solchen aus der glatten Muskulatur von Magen und Herz.

Aus den pharmakologischen Daten der vorstehenden Tabelle ergibt sich – in völliger Übereinstimmung mit den Rezeptor-Bindungs-Studien –, dass die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. «F.» bedeutet «Schmelzpunkt», «Z». bedeutet «Zersetzung».

Beispiel 1

5,11-Dihydro-11-[[(1-methyl -4- piperidinyl)-amino]carbonyl]- 6H-pyrido[2,3-b][1,4]benzodiazepin -6- on

3,6 g (0,013 Mol) 11-Chlorcarbonyl-5,11-dihydro-6H-pyrido[2,3-b][1,4]benzodiazepin -6-on und 1,4 g (0,013 Mol) Natriumcarbonat wurden in 80 ml wasserfreiem Ethanol zusammen mit 1,75 g (0,016 Mol) 4-Amino -1- methyl-piperidin 3 Stunden unter Rückfluss gekocht. Es wurde heiss filtriert, das Filtrat wurde abgekühlt und die auskristallisierte Substanz abgenutscht. Das erhaltene Rohprodukt wurde aus wasserfreiem Ethanol umkristallisiert. Farblose Kristalle vom F. 144–147 °C.

Ausbeute: 73% der Theorie.

Entsprechend erhielt man:

cis-5,11-Dihydro-11-[[1,2-dimethyl -4- piperidinyl)amino]carbonyl]- 6H-pyrido[2,3-b][1,4] benzodiazepin -6- on,

trans-5,11-Dihydro-11-[[1,2-dimethyl -4- piperidinyl)amino]carbonyl]- 6H-pyrido[2,3-b][1,4] benzodiazepin -6- on,

cis-5,11-Dihydro-11-[[1,3-dimethyl -4- piperidinyl)amino]carbonyl]- 6H-pyrido[2,3-b][1,4] benzodiazepin -6- on,

trans-5,11-Dihydro-11-[[1,3-dimethyl -4- piperidinyl)amino]carbonyl]- 6H-pyrido[2,3-b][1,4] benzodiazepin -6- on,

endo-5,11-Dihydro-11-[[(8-methyl -8- azabicyclo[3,2,1]oct -3- yl)amino]carbonyl]-6H-pyrido[2,3-b][1,4]benzodiazepin -6- on,
F. 220–222 °C (Ethanol);

exo-5,11-Dihydro-11-[[(8-methyl -8- azabicyclo[3,2,1]oct -3- yl)amino]carbonyl]-6H-pyrido[2,3-b][1,4]benzodiazepin -6- on,
F. 237–239 °C (Methanol).

Beispiel 2

5,11-Dihydro-11-[[N-methyl-N-(1-methyl -4-piperidinyl)amino]carbonyl]-6H-pyrido[2,3-b]-[1,4]
benzodiazepin -6- on

4,3 g (0,015 Mol) 4-Chlorcarbonyl-5,11-dihydro-6H-pyrido[2,3-b][1,4]benzodiazepin -6-on, 1,6 g (0,015 Mol) Natriumcarbonat und 2,56 g (0,02 Mol) 1-Methyl -4- methylamino-piperidin wurden in 150 ml wasserfreiem Ethanol 2 1/2 Stunden unter Rückfluss gerührt. Es wurde heiss filtriert, das Filtrat im Vakuum eingedampft und der Rückstand aus Essigsäureethylester und anschliessend aus Acetonitril umkristallisiert. Farblose Kristalle vom F. 230,5–232,0 °C.

Ausbeute: 54% der Theorie.

Entsprechend erhielt man:

cis-5,11- Dihydro-11- [[N-methyl- N-(1,2-dimethyl -4- piperidinyl)amino]carbonyl]-6H-pyrido[2,3-b][1,4]benzodiazepin -6- on,

trans-5,11-Dihydro-11- [[N-methyl- N-(1,2-dimethyl -4- piperidinyl)amino]carbonyl]-6H-pyrido[2,3-b][1,4]benzodiazepin -6- on,

cis-5,11-Dihydro-11- [[N-methyl- N-(1,3-dimethyl -4- piperidinyl)amino]carbonyl]-6H-pyrido[2,3-b][1,4]benzodiazepin -6- on,

trans-5,11-Dihydro-11-[[N-methyl-N-(1,3-dimethyl -4- piperidinyl)amino]carbonyl]-6H-pyrido[2,3-b][1,4]benzodiazepin -6- on,

endo-5,11-Dihydro-11-[[N-methyl-N-(8-methyl -8- azabicyclo[3,2,1]oct -3- yl)amino]carbonyl]-6H-pyrido[2,3-b] [1,4]benzodiazepin-6-on, F.277–279°C (Ethanol),

exo-5,11-Dihydro-11-[[N-methyl-N-(8-methyl -8- azabicyclo[3,2,1]oct -3- yl)amino]carbonyl]-6H-pyrido[2,3-b][1,4]benzodiazepin-6-on,

5,11-Dihydro-11-[[N-methyl- N-(4-methyl -1-piperazinyl)amino]carbonyl]- 6H-pyrido[2,3-b]-[1,4]benzodiazepin -6- on, F. 278–279 °C.

Beispiel 3

5,11-Dihydro-11-[[(4-methyl -1- piperazinyl)-amino]carbonyl]- 6H-pyrido[2,3-b][1,4]benzodiazepin -6- on

Die Mischung aus 5,5 g (0,0201 Mol) 11-Chlorcarbonyl-5,11-dihydro-6H-pyrido[2,3-b][1,4]benzodiazepin -6- on, 6,9 g (0,06 Mol) 1-Amino -4- methylpiperazin und 150 ml wasserfreiem Dioxan wurde 30 Minuten auf dem Dampfbad erwärmt, die erhaltene trübe Reaktionsmischung noch heiss mit 2 g Aktivkohle versetzt, filtriert und das erhaltene Filtrat im Vakuum eingeengt. Der Rückstand wurde an 300 g Kieselgel unter Verwendung einer Mischung aus Dichlormethan, Methanol und konz. wässrigem Ammoniak (Volumenverhältnis 800:200:5) säulenchromatographisch gereinigt. Der nach dem Eindampfen der interessierenden Eluate verbleibende Rückstand wurde aus Ethanol umkristallisiert. Man erhält 1,5 g (19% der Theorie) an farblosen Kristallen von 5,11-Dihydro-11-[[(4-methyl -1- piperazinyl)amino]carbonyl]-6H-pyrido-[2,3-b][1,4]benzodiazepin -6- on-hydrochlorid-hydrat vom F. 219–221 °C (Z.). Das Salz wurde in wenig Wasser gelöst, mit der berechneten Menge 10%iger Natronlauge alkalisch gestellt und bei einer Badtemperatur von 40 °C im Vakuum eingedampft. Der verbleibende Rückstand wurde in trockenem Tetrahydrofuran aufgenommen und filtriert, das Filtrat abermals im Vakuum vom Lösungsmittel befreit. Nach dem Umkristallisieren aus Essigsäureethylester erhielt man die gewünschte wasserlösliche, farblose Base in Form farbloser Kristalle vom F. 213–215 °C (Z.).

Entsprechend erhielt man:

5,11-Dihydro-11-[[(3,4-dimethyl -1- piperazinyl)amino]carbonyl]- 6H-pyrido[2,3-b][1,4] benzodiazepin -6- on,

5,11-Dihydro-11-[[(2,4-dimethyl -1- piperazinyl)amino]carbonyl]- 6H-pyrido[2,3-b][1,4] benzodiazepin -6- on,

endo-5,11-Dihydro-11-[[N-methyl-N-(8-methyl -8- azabicyclo[3,2,1]oct -3- yl)amino]carbonyl]-6H-pyrido[2,3-b][1,4]benzodiazepin -6-on,

5,11-Dihydro-11- [[N-methyl-N- (1-methyl -4-piperidinyl)amino]carbonyl]- 6H-pyrido[2,3-b]-[1,4]benzodiazepin -6- on, F. 230,5–232 °C (Acetonitril).

5,11-Dihydro-11-[[N-methyl-N-(3,4-dimethyl -1- piperazinyl)amino]carbonyl]-6H-pyrido[2,3-b][1,4]benzodiazepin -6- on,

5,11-Dihydro-11-[[N-methyl-N-(2,4-dimethyl -1- piperazinyl)amino]carbonyl]-6H-pyrido[2,3-b][1,4]benzodiazepin -6- on

Beispiel 4

5,11-Dihydro-11-[[(1-methyl -4- piperidinyl)-oxy]carbonyl]-6H- pyrido[2,3-b] [1,4]benzodiazepin -6- on

4,1 g (0,015 Mol) 11-Chlorcarbonyl-5,11-dihydro-6H-pyrido[2,3-b][1,4]benzodiazepin -6-on wurden mit 3,45 g (0,03 Mol) 1-Methyl -4-piperidinol in 50 ml Chlorbenzol zwei Stunden unter Rückfluss erhitzt. Nach dem Abkühlen wurde die Reaktionslösung durch Zugabe von 200 ml Essigsäureethylester verdünnt und anschliessend mit 15 prozentiger Salzsäure erschöpfend extrahiert. Man neutralisierte die vereinigten Extrakte mit Kaliumcarbonat, extrahierte mehrmals mit Chloroform und engte die vereinigten Chloroformauszüge im Vakuum zur Trockne ein. Der Rückstand wurde an Kieselgel unter Verwendung von Essigsäureethylester/Methanol (Volumenverhältnis = 1:1) zum Eluieren säulenchromatographisch gereinigt. Man erhielt farblose Kristalle vom F. 247–248 °C in einer Ausbeute von 4,0 g (76% der Theorie).

Entsprechend erhielt man:

cis-5,11-Dihydro-11-[[(1,2-dimethyl -4- piperidinyl)oxy]carbonyl]-6H-pyrido[2,3-b][1,4]benzodiazepin -6- on,

trans-5,11-Dihydro-11-[[(1,2-dimethyl -4- piperidinyl)oxy]carbonyl]-6H-pyrido[2,3-b][1,4] benzodiazepin -6- on,

cis-5,11-Dihydro-11-[[(1,3-dimethyl -4- piperidinyl)oxy]carbonyl]-6H-pyrido[2,3-b][1,4]benzodiazepin -6- on,

trans-5,11-Dihydro-11-[[(1,3-dimethyl -4-piperidinyl)oxy]carbonyl]-6H-pyrido[2,3-b][1,4] benzodiazepin -6- on,

5,11-Dihydro-11-[[(4-methyl -1- piperazinyl)-oxy]carbonyl]-6H-pyrido[2,3-b][1,4]benzodiazepin -6- on,

5,11-Dihydro-11-[[(3,4-dimethyl -1- piperazinyl)oxy]carbonyl]- 6H-pyrido[2,3-b] [1,4]benzodiazepin -6- on,

5,11-Dihydro-11-[[(2,4-dimethyl -1- piperazinyl)oxy]carbonyl]- 6H-pyrido[2,3-b] [1,4]benzodiazepin -6- on,

endo-5,11-Dihydro-11-[[(8-methyl -8- azabicyclo[3,2,1]oct -3- yl)oxy]carbonyl]-6H-pyrido[2,3-b][1,4]benzodiazepin -6- on, F. 200 °C (Essigsäureethylester)

exo-5,11-Dihydro-11-[[(8-methyl -8- azabicyclo[3,2,1]oct -3- yl)oxy]carbonyl]-6H-pyrido-[2,3-b][1,4]benzodiazepin -6- on

Beispiel 5

5,11-Dihydro-11-[[(1-methyl -4- piperidinyl)-oxy]carbonyl]- 6H-pyrido[2,3-b] [1,4]benzodiazepin -6- on

In eine Mischung, bestehend aus 22,5 ml einer 20 prozentigen Lösung von Phosgen in Toluol, 100 ml Dioxan und 4,75 g (0,045 Mol) wasserfreiem Natriumcarbonat wurden unter äusserer Kühlung mit Eis 4,9 g (0,0425) Mol) 1-Methyl -4- piperidinol zugetropft. Man rührte noch 60 Minuten bei Zimmertemperatur, trug dann 9,0 g (0,0428 Mol) 5,11-Dihydro-6H-pyrido[2,3-b]-[1,4]benzodiazepin -6- on in die Reaktionsmischung ein und kochte anschliessend 4 Stunden unter Rückfluss. Man filtrierte, dampfte das Filtrat im Vakuum ein und reinigte das erhaltene Rohprodukt säulenchromatographisch an 500 g Kieselgel unter Verwendung von Essigsäureethylester/Methanol (Volumenverhältnis 10:2) zum Eluieren. Nach dem Umkristallisieren aus Essigsäureethylester schmolzen die farblosen Kristalle bei 247–248 °C und waren nach Mischschmelzpunkt, Dünnschichtchromatogramm und IR-Spektrum identisch mit einem nach Beispiel 4 hergestellten Präparat.

Ausbeute: 5,8 g (39% der Theorie).

Entsprechend erhielt man:

5,11-Dihydro-11-[[(4-methyl -1- piperazinyl)-oxy]carbonyl]- 6H-pyrido[2,3-b] [1,4]benzodiazepin -6- on,

endo-5,11-Dihydro-11-[[(8-methyl -8- azabicyclo[3,2,1]oct -3- yl)oxy]carbonyl]-6H-pyrido[2,3-b][1,4]benzodiazepin -6- on, F. 200 °C (Essigsäureethylester)

Beispiel 6

11-Chlorcarbonyl-5,11-dihydro-6H-pyrido[2,3-b][1,4] benzodiazepin -6- on

Zu 300 ml (0,6 Mol) einer 20%igen Phosgenlösung in Toluol wurden bei Zimmertemperatur innerhalb von 2 Stunden 42 g (0,2 Mol) 5,11-Dihydro-6H-pyrido[2,3-b][1,4]benzodiazepin -6-on, gelöst in einer Mischung aus 1,5 l trockenem Dioxan und 24 ml wasserfreiem Pyridin, zugetropft. Man rührte die Mischung zwei weitere Stunden bei 60 °C, filtrierte über Aktivkohle und rührte das Filtrat in 6 l Eiswasser ein. Der ausgefallene schmierige Niederschlag wurde durch Zusatz von Ether zur Kristallisation gebracht, abgenutscht und mehrfach in Essigsäureethylester aufgeschlämmt. Nach dem Umkristallisieren aus Acetonitril schmolzen die farblosen Kristalle bei 260–261 °C.

Ausbeute: 33 g (52% der Theorie).

Entsprechend erhielt man

aus 5,11-Dihydro-6H-pyrido[2,3-b][1,4]benzodiazepin -6- on und Chlorkohlensäuremethylester in Dioxan/Toluol-Gemisch das 5,11-Dihydro-11- methoxycarbonyl- 6H-pyrido [2,3-b]-[1,4]benzodiazepin -6- on vom F. 266–267 °C (Z.) (aus 1,2-Dichlorethan);

aus 5,11-Dihydro-6H-pyrido[2,3-b][1,4]benzodiazepin -6- on und Chlorkohlensäurebenzylester in Dioxan/Toluol-Gemisch das 11-Benzyloxycarbonyl-5,11-dihydro-6H-pyrido[2,3-b][1,4]benzodiazepin -6- on;

aus 5,11-Dihydro-6H-pyrido[2,3-b][1,4]benzodiazepin -6- on und Chlorkohlensäurephenylester in Dioxan/Toluol-Gemisch das 5,11-Dihydro-11-phenyloxycarbonyl- 6H-pyrido [2,3-b]-[1,4]benzodiazepin -6- on.

Beispiel 7

5,11-Dihydro-11-[[(4-methyl -1- piperazinyl)-amino]carbonyl]- 6H-pyrido[2,3-b] [1,4]benzodiazepin -6- on-hydrochlorid-hydrat

1 g 5,11-Dihydro-11-[[(4-methyl -1- piperazinyl)- amino]carbonyl]- 6H- pyrido [2,3-b]-[1,4]benzodiazepin -6- on wurde in wenig Ethanol gelöst mit ethanolischer Salzsäure versetzt. Es wurde weitgehend eingeengt und mit Aceton versetzt und der Niederschlag aus Ethanol umkristallisiert. Es wurden 0,8 g 5,11-Dihydro-11-[[(4-methyl -1- piperazinyl)amino]carbonyl]- 6H-pyrido [2,3-b] [1,4]benzodiazepin- 6-on-hydrochlorid-hydrat erhalten;
F. 219–221 °C (Z.).

Analog wurden erhalten:
5,11-Dihydro-11-[[(4-methyl -1- piperazinyl)-amino]carbonyl]- 6H-pyrido[2,3-b][1,4]benzodiazepin -6- on-hydrobromid-hydrat;
F. 212–213 °C (Z.);
5,11-Dihydro-11-[[(4-methyl -1- piperazinyl)-amino]carbonyl]- 6H-pyrido[2,3-b][1,4]benzodiazepin -6- on-maleinat-hydrat;
F. 152–153 °C (Z.);
5,11-Dihydro-11-[[(4-methyl -1- piperazinyl)-amino]carbonyl]- 6H-pyrido[2,3-b][1,4]benzodiazepin -6- on-hemifumarat-hydrat;
F. 159–160 °C (Z.);
5,11-Dihydro-11-[[(4-methyl -1- piperazinyl)-amino]carbonyl]- 6H-pyrido[2,3-b][1,4]benzodiazepin -6- on-citrat; F. 131–133 °C (Z.);
5,11-Dihydro-11-[[(4-methyl -1- piperazinyl)-amino]carbonyl]- 6H-pyrido[2,3-b][1,4]benzodiazepin -6- on-methansulfonat;
F. 239–240 °C (Z.);
5,11-Dihydro-11-[[(4-methyl -1- piperazinyl)-amino]carbonyl]- 6H-pyrido[2,3-b][1,4]benzodiazepin -6- on-sulfat-hydrat, F. 192–193 °C (Z.);
5,11-Dihydro-11-[[(4-methyl -1- piperazinyl)-amino]carbonyl]- 6H-pyrido[2,3-b][1,4]benzodiazepin -6- on-hemitartrat-dihydrat;
F. 165–166 °C (Z.);
5,11-Dihydro-11-[[(4-methyl -1- piperazinyl)-amino]carbonyl]- 6H-pyrido[2,3-b][1,4]benzodiazepin -6- on-phosphat-dihydrat;
F. 280–281 °C (Z.).

Beispiel 8

5,11-Dihydro-11-[[(4-methyl -1- piperazinyl)-amino]carbonyl]- 6H-pyrido[2,3-b][1,4]benzodiazepin -6- on-hydrochlorid-hydrat

Zu der Suspension von 73,5 g (0,269 Mol) 11-Chlorcarbonyl- 5,11- dihydro- 6H- pyrido-[2,3-b][1,4]benzodiazepin -6- on in 200 $cm^3$ Dimethylformamid tropft man unter Rühren innerhalb von etwa 1 Stunde die Lösung von 31,7 g (0,275 Mol) 1-Amino -4- methylpiperazin in 50 $cm^3$ Dimethylformamid. Durch äussere Kühlung mit Eis oder Eiswasser reguliert man die Reaktionstemperatur auf nicht viel oberhalb 0 °C, maximal 5 °C, ein und hält anschliessend noch 1 Stunde bei dieser Temperatur. Nach dem Erwärmen auf Zimmertemperatur versetzt man die Mischung mit 500 $cm^3$ Ethanol und impft mit einer Probe von 5,11-Dihydro-11-[[(4-methyl -1- piperazinyl)amino]carbonyl]- 6H-pyrido[2,3-b]-[1,4]benzodiazepin -6- on-hydrochlorid-hydrat (aus früheren Ansätzen!) an. Man rührt bis zur vollständigen Ausfällung des Hydrochlorids (ca. 1 Stunde), nutscht den Niederschlag ab und wäscht ihn mit 200 $cm^3$ Ethanol aus. Man kristallisiert aus 700 $cm^3$ einer siedendheissen Mischung von Ethanol und Methanol (80:20 v/v) unter Verwendung von 3 g Aktivkohle um. Die zunächst erhaltene Ausbeute an gewünschtem Produkt von 44 g kann durch Eindampfen der Mutterlaugen auf ca. 200 $cm^3$ um weitere 28 g gleicher Reinheit gesteigert werden. Man trocknet im Vakuum bei 140 °C und lässt anschliessend über Nacht offen an der Luft stehen zwecks Bildung des Monohydrats. F. 219–221 °C (Z.),

Gesamtausbeute: 72,0 g (66% der Theorie).

Im folgenden wird die Herstellung pharmazeutischer Anwendungsformen anhand einiger Beispiele beschrieben:

Beispiel I

Tabletten mit 5 mg 5,11-Dihydro -4-[[(4-methyl-1- piperazinyl)amino]carbonyl]-6H-pyrido[2,3-b][1,4]benzodiazepin -6- on

Zusammensetzung:
1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpresst.

| | |
|---|---|
| Tablettengewicht: | 220 mg |
| Stempel: | 9 mm |

Beispiel II

Dragées mit 5 mg 5,11-Dihydro -4- [[(4-methyl - 1- piperazinyl)amino]carbonyl]- 6H-pyrido[2,3-b][1,4]benzodiazepin -6- on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Ampullen mit 1 mg 5,11-Dihydro -4- [[(4-methyl -1- piperazinyl)amino]carbonyl]-6H-pyrido[2,3-b][1,4]benzodiazepin -6- on

Zusammensetzung:
1 Ampulle enthält:

| | |
|---|---|
| Wirkstoff | 1,0 mg |
| Natriumchlorid | 8,0 mg |
| Dest. Wasser ad | 1 ml |

Herstellungsverfahren:

Die Wirksubstanz und Natriumchlorid werden in dest. Wasser gelöst und anschliessend auf das

gegebene Volumen aufgefüllt. Die Lösung wird sterilfiltriert und in 1 ml-Ampullen abgefüllt.
Sterilisation: 20 Minuten bei 120 °C.

Beispiel IV

Suppositorien mit 5 mg 5,11-Dihydro -4- [[(4-methyl -1- piperazinyl)amino]carbonyl]-6H-pyrido[2,3-b] [1,4]benzodiazepin -6- on

Zusammensetzung:
1 Zäpfchen enthält:

| | |
|---|---|
| Wirkstoff | 5,0 mg |
| Zäpfchenmasse (z.B. Witepsol W 45[R]) | 1 695,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man giesst die Masse bei 37 °C in leicht vorgekühlte Zäpfchenformen aus.
Zäpfchengewicht: 1,7 g

Beispiel V

Tropfen mit 5,11-Dihydro -4- [[(4-methyl -1- piperazinyl)amino]carbonyl]- 6H-pyrido[2,3-b]-[1,4]benzodiazepin -6- on enthaltend 0,5 g Wirkstoff/100 ml Lösung

Zusammensetzung:
100 ml Tropflösung enthalten:

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wässrigen Lösung zu. Abschliessend wird mit Wasser auf 100 ml aufgefüllt und schwebeteilchenfrei filtriert.

**Patentansprüche**

1. Pyridobenzodiazepinone der allgemeinen Formel I

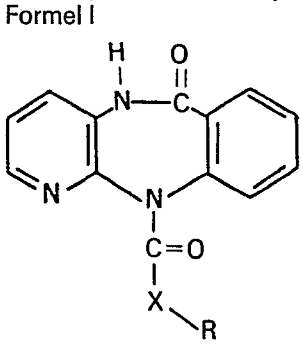

(I)

in der

X ein Sauerstoffatom, eine –NH- oder –$NCH_3$-Gruppe und

R einen gegebenenfalls durch eine Methylgruppe substituierten 1-Methyl -4- piperidinyl-, 4-Methyl -1- piperazinyl-, 3α- oder 3β-Tropanylrest bedeuten, gegebenenfalls ihre diastereomeren und enantiomeren Formen und ihre physiologisch verträglichen Säureadditionssalze mit anorganischen oder organischen Säuren.

2. Pyridobenzodiazepinone der allgemeinen Formel I gemäss Anspruch 1, in der

X ein Sauerstoffatom oder eine –NH-Gruppe und

R eine 1-Methyl -4- piperidinyl- oder 4-Methyl -1- piperazinylgruppe bedeuten, und ihre physiologisch verträglichen Säureadditionssalze mit anorganischen oder organischen Säuren.

3. 5,11-Dihydro-11-[[(4-methyl -1- piperazinyl)amino]carbonyl]-6H-pyrido[2,3-b] [1,4] benzodiazepin -6- on und dessen physiologisch verträgliche Säureadditionssalze.

4. Arzneimittel, enthaltend eine Verbindung gemäss den Ansprüchen 1 bis 3 neben einem oder mehreren inerten Trägerstoffen und/oder Verdünnungsmitteln.

5. Verfahren zur Herstellung von Pyridobenzodiazepinonen der allgemeinen Formel I

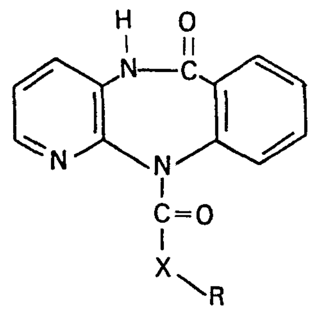

(I)

in der

X ein Sauerstoffatom, eine –NH- oder –$NCH_3$-Gruppe und

R einen gegebenenfalls durch eine Methylgruppe substituierten 1-Methyl -4- piperidinyl-, 4-Methyl -1- piperazinyl-, 3α- oder 3β-Tropanylrest bedeuten, von deren diastereomeren und enantiomeren Formen und von ihren Säureadditionssalzen mit anorganischen oder organischen Säuren, dadurch gekennzeichnet, dass

a) ein Pyridobenzodiazepinon der allgemeinen Formel Ia

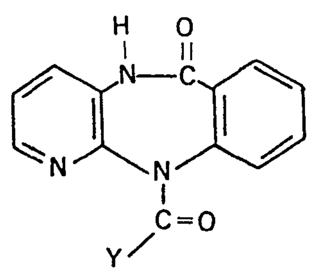

(Ia)

in der

Y ein Halogenatom, vorzugsweise ein Chlor- oder Bromatom, oder die Gruppe $OR_1$ bedetuet, worin $R_1$ einen gegebenenfalls halogensubstituierten Alkylrest mit 1 bis 5 Kohlenstoffatomen,

einen gegebenenfalls durch Halogen oder Nitro substituierten Phenylrest oder einen Aralkylrest mit 7 bis 15 Kohlenstoffatomen darstellt, mit einer Verbindung der allgemeinen Formel

H–X–R (II)

oder

M–X–R (IIa)

in denen

R und X wie oben angegeben definiert sind und M ein Alkalimetallatom oder 1 Äquivalent eines Erdalkalimetallatoms bedeutet, gegebenenfalls in Gegenwart eines Lösungsmittels bei Temperaturen zwischen 0 °C und dem Siedepunkt des Reaktionsgemisches umgesetzt wird oder

b) 5,11-Dihydro-6H-pyrido[2,3-b][1,4]benzodiazepin -6- on mit einem Chlorkohlensäurederivat der allgemeinen Formel

Cl–C–X–R (IV)
  ||
  O

bzw. mit einem Isocyanat der allgemeinen Formel

O = C = N–R (IVa)

in denen

R und X wie oben angegeben definiert sind, gegebenenfalls in organischen Lösungsmitteln und gegebenenfalls in Gegenwart von tertiären organischen Basen, bei Temperaturen zwischen 30 °C und dem Siedepunkt des Reaktionsgemisches umgesetzt wird, und gegebenenfalls eine so erhaltene Verbindung der allgemeinen Formel I anschliessend in ihre Salze mit anorganischen oder organischen Säuren übergeführt wird.

6. Verfahren gemäss Anspruch 5a, dadurch gekennzeichnet, dass die Pyridobenzodiazepinone der allgemeinen Formel Ia mit den Verbindungen der allgemeinen Formel II in Gegenwart von Wasser, Toluol, von Alkoholen, vorzugsweise in Gegenwart aprotischer polarer Lösungsmittel oder in Gemischen solcher Lösungsmittel bei Temperaturen von 40 bis 100 °C und in Gegenwart anorganischer oder organischer Basen oder in Gegenwart eines Überschusses der Verbindungen der allgemeinen Formel II zur Reaktion gebracht werden.

7. Verfahren gemäss Anspruch 5b, dadurch gekennzeichnet, dass die Umsetzung in aromatischen Kohlenwasserstoffen, in Acetonitril, Dimethylformamid oder in Gemischen dieser Lösungsmittel bei Temperaturen zwischen 30 und 100 °C erfolgt.

8. Pyridobenzodiazepinone der allgemeinen Formel Ia

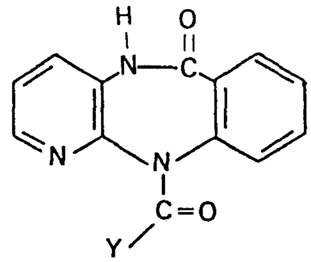

(Ia)

in der

Y ein Halogenatom, vorzugsweise ein Chlor- oder Bromatom, oder die Gruppe $OR_1$ bedeutet, worin $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt.

9. Verfahren zur Herstellung von Pyridobenzodiazepinonen der allgemeinen Formel Ia

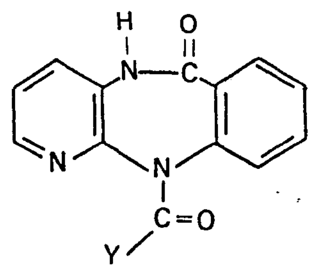

(Ia)

in der

Y ein Halogenatom, vorzugsweise ein Chlor- oder Bromatom, oder die Gruppe $OR_1$ bedeutet, worin $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, dadurch gekennzeichnet, dass 5,11-Dihydro-6H-pyrido[2,3-b][1,4]benzodiazepin -6- on mit einem Halogenkohlensäurederivat der allgemeinen Formel III

   O
   ||
Hal–C–Y (III)

in der

Y wie eingangs definiert ist und

Hal ein Chlor- oder Bromatom darstellt, umgesetzt wird.

10. Verfahren gemäss Anspruch 9, dadurch gekennzeichnet, dass die Umsetzung in Gegenwart einer tertiären organischen Base und/oder eines Lösungsmittels und bei Temperaturen bis zur Siedetemperatur des verwendeten Lösungsmittels, vorzugsweise bei Temperaturen zwischen 30 und 80 °C, durchgeführt wird.

**Revendications**

1. Pyridobenzodiazépinones de formule générale I:

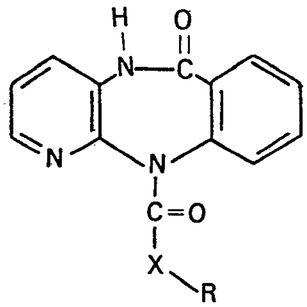

(I)

dans laquelle:

X représente un atome d'oxygène, un groupe –NH– ou –$NCH_3$ et

R un radical 1-méthyl -4- pipéridinyle, 4-méthyl -1- pipérazinyle, 3α- ou 3β-tropanyle, éventuellement substitué par un groupe méthyle, le cas échéant ses formes diastéréo-isomères et énantiomères et ses sels d'addition d'acides compatibles physiologiquement avec des acides organiques ou minéraux.

2. Pyridobenzodiazépinones de formule générale I selon la revendication 1, dans laquelle:

X représente un atome d'oxygène ou un groupe –NH– et

R un groupe 1-méthyl -4- pipéridinyle ou 4-méthyl -1- pipérazinyle, et leurs sels d'addition d'acides compatibles physiologiquement avec des acides organiques ou minéraux.

3. La 5,11-dihydro-11-[[(4-méthyl -1- pipérazinyl)- amino]carbonyl]- 6H- pyrido [2,3-b]-[1,4]benzodiazépine -6- one et ses sels d'addition d'acides compatibles physiologiquement.

4. Médicament contenant un composé selon les revendications 1 à 3 conjointement à un ou plusieurs excipients et/ou diluants inertes.

5. Procédé pour la préparation de nouvelles pyridobenzodiazépinones de formule générale I:

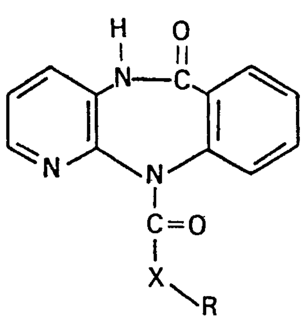

(I)

dans laquelle:

X représente un atome d'oxygène, un groupe –NH– ou –$NCH_3$ et

R un radical 1-méthyl -4- pipéridinyle, 4-méthyl -1- pipérazinyle, 3α- ou 3β-tropanyle, éventuellement substitué par un groupe méthyle, de ses formes diastéréo-isomères et énantiomères et de ses sels d'addition d'acides avec des acides organiques ou minéraux, caractérisé en ce que

a) on fait réagir une pyridobenzodiazépinone de formule générale Ia:

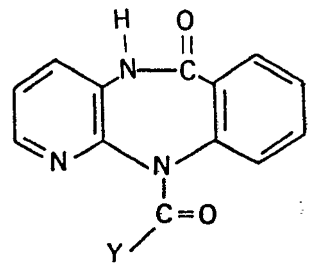

(Ia)

dans laquelle:

Y représente un atome d'halogène, de préférence un atome de chlore ou de brome, ou bien représente le groupe $OR_1$, dans lequel $R_1$ représente un radical alcoyle avec 1 à 5 atomes de carbone éventuellement halogéno-substitué, un radical phényle éventuellement substitué par halogène ou nitro, ou un radical aralcoyle avec 7 à 15 atomes de carbone, avec un composé de formule générale:

$$H-X-R \quad (II)$$

ou

$$M-X-R \quad (IIa)$$

dans lesquelles:

R et X sont tels que définis ci-dessus et M représente un atome de métal alcalin ou un équivalent d'un atome de métal alcalino-terreux, éventuellement en présence d'un solvant à des températures entre 0 °C et le point d'ébullition du mélange réactionnel ou

b) on fait réagir la 5,11-dihydro-6H-pyrido[2,3-b][1,4]benzodiazépine -6- one avec un dérivé d'acide chlorocarbonique de formule générale:

$$Cl-\underset{\underset{O}{\|}}{C}-X-R \quad (IV)$$

ou respectivement avec un isocyanate de formule générale:

$$O=C=N-R \quad (IVa)$$

dans lesquelles:

R et X sont tels que définis ci-dessus, le cas échéant dans des solvants organiques et éventuellement en présence de bases organiques tertiaires, à des températures comprises entre 30 °C et le point d'ébullition du mélange réactionnel, et éventuellement un composé ainsi obtenu de formule générale I est ensuite transformé en ses sels avec des acides organiques ou minéraux.

6. Procédé selon la revendication 5a, caractérisé en ce que les pyridobenzodiazépinones de formule générale Ia sont mises en réaction avec les composés de formule générale II en présence d'eau, de toluène, d'alcools, de préférence en présence de solvants polaires aprotiques ou dans des mélanges de tels solvants à des températures de 40 à 100 °C et en présence de bases organiques ou

minérales ou en présence d'un excédent des composés de formule générale II.

7. Procédé selon la revendication 5b, caractérisé en ce que la réaction dans les hydrocarbures aromatiques, dans l'acétonitrile, le diméthylformamide ou dans les mélanges de ces solvants s'effectue à des températures entre 30 et 100 °C.

8. Pyridobenzodiazépinones de formule générale Ia:

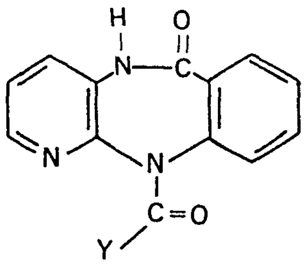

(Ia)

dans laquelle:

Y représente un atome d'halogène, de préférence un atome de chlore ou de brome, ou bien le groupe $OR_1$, dans lequel $R_1$ représente un radical alcoyle éventuellement halogéno-substitué avec 1 à 5 atomes de carbone, un radical phényle éventuellement substitué par halogène ou nitro, ou un groupe aralcoyle avec 7 à 15 atomes de carbone.

9. Procédé pour la préparation de pyridobenzodiazépinones de formule générale Ia:

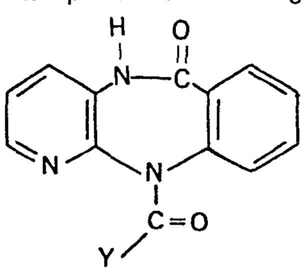

(Ia)

dans laquelle:

Y représente un atome d'halogène, de préférence un atome de chlore ou de brome, ou bien le groupe $OR_1$, dans lequel $R_1$ représente un radical alcoyle éventuellement halogéno-substitué avec 1 à 5 atomes de carbone, un radical phényle éventuellement substitué par halogène ou nitro, ou un groupe aralcoyle avec 7 à 15 atomes de carbone, caractérisé en ce qu'on fait réagir la 5,11-dihydro-6H-pyrido[2,3-b][1,4]benzodiazépine -6- one avec un dérivé d'acide carbonique halogéné de formule générale III:

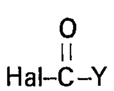

(III)

dans laquelle:

Y est tel que défini au départ et

Hal représente un atome de chlore ou de brome.

10. Procédé selon la revendication 9, caractérisé en ce que la réaction est effectuée en présence d'une base organique tertiaire et/ou d'un solvant et à des températures jusqu'au point d'ébullition du solvant utilisé, de préférence à des températures entre 30 et 80 °C.

**Claims**

1. Pyridobenzodiazepinones of general formula I,

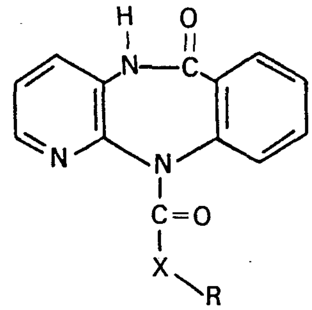

(I)

wherein

X represents an oxygen atom or an –NH– or $-NCH_3$-group and

R represents a 1-methyl -4- piperidinyl, 4-methyl -1- piperazinyl or 3α- or 3β-tropanyl group, each of the groups being optionally substituted by a further methyl group, and optionally the diastereo-meric and enantiomeric forms thereof and the physio-logically acceptable acid addition salts thereof with inorganic or organic acids.

2. Pyridobenzodiazepinones of general formula I as claimed in claim 1, wherein

X represents an oxygen atom or an –NH– group and

R represents the 1-methyl -4- piperidinyl or 4-methyl -1- piperazinyl group, and the physiologically acceptable acid addition salts thereof with inorganic or organic acids.

3. 5,11-dihydro-11-[[(4-methyl -1- piperazinyl)amino]carbonyl]- 6H-pyrido[2,3-b][1,4] benzodiazepin -6- one and the physiologically acceptable acid addition salts thereof.

4. Pharmaceutical compositions containing a compound as claimed in claims 1 to 3 together with one or more inert carriers and/or diluents.

5. Process for the preparation of pyridobenzodiazepinones of general formula I

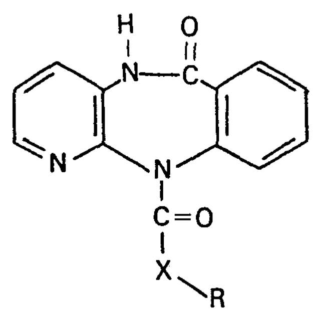

(I)

wherein

X represents an oxygen atom or an –NH– or $-NCH_3$-group and

R represents a 1-methyl -4- piperidinyl, 4-methyl -1- piperazinyl or 3α- or 3β-tropanyl group, each of the groups being optionally substituted by a further methyl group, and of the diastereomeric and enantiomeric forms thereof and of the acid addition salts thereof with inorganic or organic acids, characterised in that

a) a pyridobenzodiazepinone of general formula Ia,

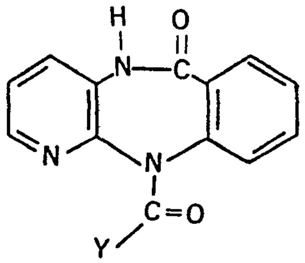

(Ia)

wherein

Y represents a halogen atom, preferably a chlorine or bromine atom, or the group $OR_1$ wherein $R_1$ represents an optionally halogen-substituted alkyl group with 1 to 5 carbon atoms, a phenyl group optionally substituted by halogen or nitro or an aralkyl group with 7 to 15 carbon atoms, is reacted with a compound of general formula

H–X–R (II)

or

M–X–R (IIa)

wherein

R and X are as hereinbefore defined and M represents an alkali metal atom or one equivalent of an alkaline earth metal atom, optionally in the presence of a solvent at temperatures of between 0 °C and the boiling point of the reaction mixture or

b) 5,11-dihydro-6H-pyrido[2,3-b][1,4]benzodiazepin -6- one is reacted with a chlorocarbonic acid derivative of general formula

Cl–C–X–R (IV)
  ||
  O

or with an isocyanate of general formula

O =C =N–R (IVa)

wherein

R and X are as hereinbefore defined, optionally in organic solvents and optionally in the presence of tertiary organic bases, at temperatures of between 30°C and the boiling point of the reaction mixture, and if desired a compound of general formula I thus obtained is subsequently converted into the salts thereof with inorganic or organic acids.

6. Process as claimed in claim 5a, characterised in that the pyridobenzodiazepinones of general formula Ia are reacted with the compounds of general formula II in the presence of water, toluene, alcohols, preferably in the presence of aprotic polar solvents or in mixtures of such solvents at temperatures of from 40 to 100 °C and in the presence of inorganic or organic bases or in the presence of an excess of the compounds of general formula II.

7. Process as claimed in claim 5b, characterised in that the reactions carried out in aromatic hydrocarbons, in acetonitrile, dimethylformamide or in mixtures of these solvents at temperatures between 30 and 100°C.

8. Pyridobenzodiazepinones of general formula Ia,

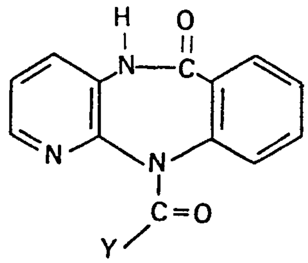

(Ia)

wherein

Y represents a halogen atom, preferably a chlorine or bromine atom, or the group $OR_1$, wherein $R_1$ represents an optionally halogen-substituted alkyl group with 1 to 5 carbon atoms, a phenyl group optionally substituted by halogen or nitro or an aralkyl group with 7 to 15 carbon atoms.

9. Process for the preparation of new pyridobenzodiazepinones of general formula Ia,

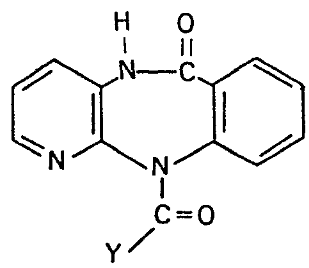

(Ia)

wherein

Y represents a halogen atom, preferably a chlorine or bromine atom, or the group $OR_1$, wherein $R_1$ represents an optionally halogen-substituted alkyl group with 1 to 5 carbon atoms, a phenyl group optionally substituted by halogen or nitro or an aralkyl group with 7 to 15 carbon atoms, characterised in that 5,11-dihydro-6H-pyrido[2,3-b][1,4]benzodiazepin -6- one is reacted with a halocarbonic acid derivative of general formula III

    O
    ||
Hal–C–Y (III)

wherein

Y is as hereinbefore defined and

Hal represents a chlorine or bromine atom.

10. Process as claimed in claim 9, characterised in that the reaction is carried out in the presence of a tertiary organic base and/or a solvent and at temperatures up to the boiling temperature of the solvent used, preferably at temperatures of between 30 and 80°C.